## Europäisches Patentamt
## European Patent Office
## Office européen des brevets

(19)

(11) Publication number: **0 001 933**
**A2**

(12)

# EUROPEAN PATENT APPLICATION

(21) Application number: **78300602.6**

(22) Date of filing: **07.11.78**

(51) Int. Cl.²: **G 01 S 7/62**
**A 61 B 10/00**

(30) Priority: **07.11.77 US 848987**

(43) Date of publication of application:
**16.05.79 Bulletin 79/10**

(84) Designated contracting states:
**DE FR GB NL**

(71) Applicant: **LITTON INDUSTRIAL PRODUCTS INC.**
**360 North Crescent Drive**
**Beverly Hills California 90210(US)**

(72) Inventor: **Cribbs, Robert**
**Lake View Drive**
**Placerville California(US)**

(72) Inventor: **Mahony, John**
**8910A Salmon Falls Drive**
**Sacramento California(US)**

(74) Representative: **Godsill, John Kenneth et al,**
**Haseltine Lake & CO. Hazlitt House 28 Southampton**
**Buildings Chancery Lane**
**London WC2A 1AT(GB)**

(54) **Video A-trace display system for ultrasonic diagnostic equipment.**

(57) A video A-trace display system for ultrasonic diagnostic equipment provides means (250) for writing pulse reflection data into a memory (111), and means (226) for selectively accessing the memory (111) to place the data on a video screen in graphic form. The data is stored and accessed in a format which is compatible with the scan pattern associated with the video display. One each video line, the accessed data is compared with a value corresponding to the ordinate value of the line to control the intensity of the beam in scanning the line.

FIG 2B.

1

## VIDEO A-TRACE DISPLAY SYSTEM FOR ULTRASONIC DIAGNOSTIC EQUIPMENT

This invention relates to ultrasonic diagnostic equipment such as that used in the medical field and, more particularly, to the displaying of the ultrasonically derived information. More specifically, this invention concerns apparatus for the video displaying of the A-trace of ultrasonic diagnostic equipment.

The A-trace is the graphically-depicted information derived from the ultrasonic pulse-echo emerging from the examined body. As is known in the art, pulse reflections occur when the ultrasonic transmitted pulse, transmitted into the examined body via a transducer, encounters a discontinuity in media defining the propogation path. The magnitude of the reflection is proportional to the difference in the media densities. The echo amplitude is typically graphically represented as the ordinate value, while the echo return time (indicative of body depth) is reproduced on the abscissa. Accordingly, one may, by viewing the A-trace, determine the depth into the body of each discontinuity in the pulse-propogation path and the type of media transition. With one's knowledge of the theoretical internal structure of the body (or, in the case of medical diagnostics, the human physiology) one may identify the locations of various structural components (or organs).

Conventional ultrasonic systems have employed cathode-ray tubes as the A-trace display means. Earlier systems employed two CRTs, the second of which was dedicated to the display of images derived by subjecting the pulse-echo signal to one of several known signal-processing techniques. The CRT possessed several inherent weaknesses, however, which led to the subsequent use of TV monitors for displaying the processed image. First, CRTs offered poor display visability. Secondly, CRTs having storage capability were expensive. Thirdly, CRTs cannot provide highly desirable alphanumeric information, such as patient-identifying data. Additionally, the use of a TV monitor permits the use of video tape, and the ability to drive several monitors from a single source, both of which are economically beneficial. The A-trace, however, continued to be displayed on a CRT, necessitating the use of two display devices. If permanently recorded images were desired, two cameras, or alternately, a single relocatable camera, were necessary.

The present invention is a video A-trace system which enables the limitations set forth above to be overcome and is for use with ultrasonic diagnostic equipment of the type including means for launching an ultrasonic pulse into a body to be examined, means for detecting emerging pulse reflections from the body and responsive thereto to produce respective amplitude-indicative electrical signals, and video display means including means for repeatedly scanning a plurality of generally parallel video lines of the display means to define a multi-lined video field.

According to one aspect of the present invention the video A-trace display system comprises:

memory means (111) having uniquely addressable locations for storing digital values;

means (250) for storing in a predetermined succession of locations of the memory means respective digital values indicative of pulse-reflection amplitudes at respective time-intervals subsequent to the launching of a pulse;

means (200,226) responsive to the initiating of a video line for accessing successive memory locations in a sequence which permits retrieval of the digital values in a reconstructed time-phase relationship; means (320) for producing a reference signal (315) having a value which is substantially proportional to the position within the video field of the video line being scanned; and means (300) for comparing the digital values accessed with the reference signal to produce video signals respectively indicative of whether the successive digital values are greater than or less than the reference signal.

According to another aspect of the invention, the video A-trace display system comprises:

first timing means responsive to the launching of an ultrasonic pulse for producing a first series of timing pulses (LCLK);

second timing means responsive to the commencement of a video line to produce a second series of timing pulses (DCLK);

memory means (111) having uniquely addressed locations for storing digital values;

means (104) responsive to said first timing means to sample the amplitude-indicative signals at successive discrete times subsequent to the pulse launching and to apply the successively sampled signals as digital data to the memory means (111);

storing means (250) responsive to the first timing means for successively accessing the address locations to permit the storing of the successively sampled data in respective memory locations;

means (200,226) responsive to the second timing means for successively accessing at least a portion of the locations accessed by the storing means, said portion being accessed during each video line scan;and means (300) responsive to the accessed data values for producing video signals.

For a better understanding of the invention and to show how the same may be carried into effect, reference will now be made, by way of example, to the accompanying drawings, in which:

Figures 1 A to C give a pictorial representation of video A-trace displays;

Figures 2 A, B and C give a block diagram of a video A-trace system; and

Figures 3A, 3B, 4A, 4B, 5A, 5B, 6A and 6B are schematic representations showing the preferred embodiment of the system in greater detail.

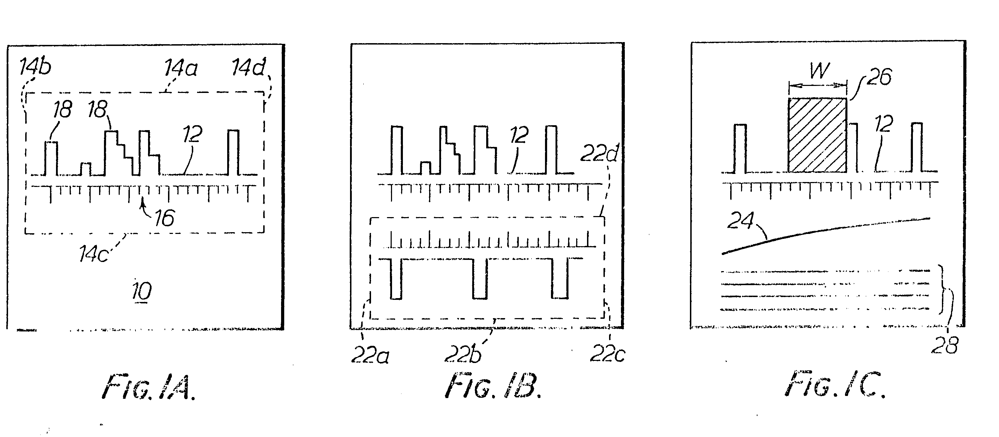

Figures 1A to 1C give a pictorial representation of video displays of an ultrasonic reflection A-trace which can be provided using techniques employed in the system hereinafter described. Figures 1A to C show a video viewing screen 10 having an A-trace 12 displayed thereon within a display area defined by dotted lines 14$\underline{a}$ to $\underline{d}$. A comb marker 16 forms the abscissa of the A-trace and denotes return time or, correspondingly, the depth into the body of the propogation path discontinuity which generated the reflection. The amplitude of the reflection is represented by the peak value of the pulses 18.

Additionally one can display on the video screen a caliper 26 (Figure 1C) the horizontal position and width W of which on the screen may be adjusted as hereinafter described by an operator so as to accurately measure the pulse width of any of the reflections 18. The measured widths may be visually displayed within several lines of alpha numeric characters 28 (Figure 1C) and conveniently placed at the bottom of the viewing screen.

Also one may display a graphic representation of the Time Gain Control curve 24 (Figure 1C). The Time Gain Control curve, or TGC curve, is a graphic representation of the time-dependent gain of the ultrasonic receiver which increases with increasing return time to compensate for the attenuation of late arriving reflections from deep within the body.

0001933

Furthermore, two successive traces, (taken for example from opposite sides of the brain) can be simultaneously displayed (Figure 1B), one being stored and then displayed in inverted form during the display of the other.

The foregoing displays have not heretofore been presented on standard video displays. During the following description, it is convenient to assume that the video displays utilize the standard raster scanning pattern known in the art, although it will be obvious that other scanning patterns may be equally utilized. However, the use of the standard raster pattern allows use of standard peripheral devices such as video tape recorders, video discs, etc. Before turning to the detailed description of the video A-trace display system, it will be helpful for future reference to note that the display boundaries 14a to d define a window within which the A-trace is displayed. This window has boundaries which are offset from the edges of the viewing screen for visual clarity; the beginning of the A-trace is thus offset from the initial position of the scanning beam just subsequent to its retrace.

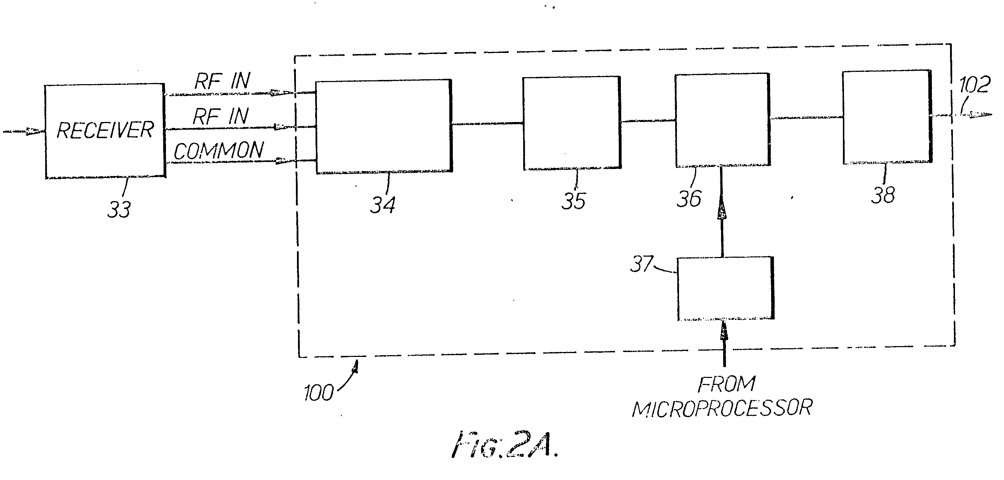

Figure 2 is a block diagram of the video A-trace system of the present invention. For the sake of clarity means for generating the various timing signals have been omitted, since many such circuits are known in the art and are peripheral to the present discussion.

The incoming echos are first quantized by level and converted to a digital form by means of a video analog-to-digital converter 100 (Figure 2A). The quantizing levels are determined by pre-programmed values from a microprocessor and which may be varied by the user. The output signal 102 is provided by a plurality of output lines which respectively indicate whether successively sampled instantaneous levels of the echo are higher or lower than the pre-programmed values.

Further details concerning the video analog-to-digital converter may be found in our co-pending Patent Application, entitled "Ultrasonic Diagnostic Equipment", filed concurrently herewith and hereby incorporated by reference.

In general, that equipment comprises a consol having a set of switches by which an operator can select any of a number of desired video processing techniques, including the A-mode and including various dynamic ranges for the A-mode. Data concerning each technique is stored in the memory of a microprocessor and the appropriate data is accessed by the operator actuating the set of switches. The accessed data control the equipment to produce the required video response.

Incoming signals are differentially fed by an ultrasonic receiver 33 to a differential-to-single-end converter 34 the output of which passes through a buffer 35 to the inverting terminal of a plurality of comparators 36. The comparators track the level of the output of the buffer 35 and determined when it is greater than respective set points or values held in sample-and-hold circuits 37. The values are fed to the non-inverting inputs of the comparators. The values concerned are obtained from the microprocessor memory as part of the data accessed by operator actuation of the set of switches. The output from the comparators comprises a plurality of binary bits forming a word held in output buffers 38.

The digitized signals thus produced are fed to the video A-trace converter, hereinafter described, which encodes the signal into a data format suitable for storage in memory, writes the data into a high speed memory, and reads the data out of memory at a speed suitable for video display.

Returning now to the A-trace converter, the period between launched pulses is divided into a number of intervals by first timing means the output of which is represented in the Figures as LCLK. The number of intervals

0001933

is a function of scale factor and may typically be 400. At each clock pulse, the peak level of the return echo during the previous clock period is sampled and written into a unique memory address. The address is incremented and the operation repeated. After the A-trace has been digitized and stored, it is possible to read the information out of memory at a different rate by incrementing the address and accessing the memory at a different speed. Accordingly, second timing means the output of which is hereinafter referred to as DCLK, is provided for reading the data out of memory. The operation and configuration of the presently disclosed embodiment is more easily explained by first considering the writing of data into memory and then considering the reading of data for display.

Figures 2B and 2C show a block diagram of the video A-trace converter of the present invention and may be utilized in conjunction with the remaining figures to obtain a general overview. As shown therein, and in more detail in Figures 3A and 3B, the quantized signals 102 from the video analog-to-digital converter are sampled by input latch means 104 at a rate determined by LCLK. In practice, twelve quantizing levels and, consequently, twelve input latches are employed, although only six are shown in Figure 3A for clarity. The pulse rate of LCLK is correlated with the depth of pulse penetration into the body, and is conveniently set at 1 pulse/mm. A change in pulse rate to 1 pulse/0.5 mm. would thereby result in a magnification of the A-trace where greater detail is necessary. The sampled values are clocked into priority encoders 106 which convert the twelve bits to a four-bit, binary-coded, data word 108 which indicates the number of active lines 102. The four-bit word 108, representing the echo amplitude value, is then clocked into latch means 110 which holds word 108 for writing into memory at the appropriate time.

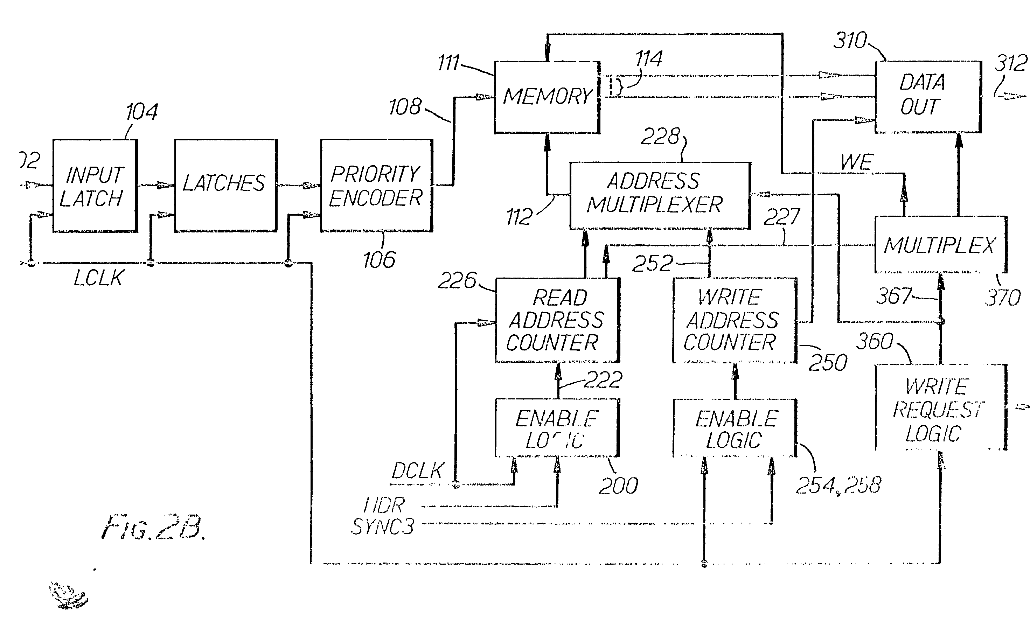

Memory    means 111 (Figure 3B) is a random access memory array which receives address signals over lines 112 and passes output data over lines 114. At each LCLK pulse, the peak reflection value during the preceeding interval is written as data into memory. By addressing the memory in a predetermined sequence, the data will be stored, for later access, in a manner by which the video elements of each video line will be correctly scanned to provide either black or white spots forming, in total, the A-trace. The manner by which address information is generated may be more readily appreciated by reference to Figures 4A and 4B in conjunction with Figure 1.

Figures 4A and 4B show the preferred address-generating circuitry generally indicated in Figure 2B. READ and WRITE addresses are separately generated so that the respective accessing rates may be independently optimized. Turning first to the WRITE address, the object is to permit the writing into memory of an entire video line at a time for the subsequent displaying of that line on the screen of a video monitor. As shown generally in Figure 2, and in more detail in Figures 4A and 4B, the WRITE address circuitry comprises counter-enabling logic 254 and 258 and address counters 250. The counter-enabling logic 254 and 258 functions to continuously clear the WRITE counters 250 until the presence of a SYNC 3 signal, indicative of the presence of data to be written into memory, is applied to the triggering input of a one-shot multi-vibrator 254. The inverted output of the one-shot multivibrator 254 clears a flip-flop 258 which, in turn, ceases the clearing of counters 250a to c. The counters 250a to c then count towards their terminal count at a clock rate determined by LCLK. The address-representative output bits 252 from the counters 250 are applied to one set of input terminals $O_A$ to $O_D$ of an address multiplexer 228 which is responsive to a mode-select signal 367 to select the WRITE addresses as its output 112a to h. Accordingly, each sampled value of the returning echo is assigned a unique memory address in a pre-arranged order for later access during the READ sequence hereinafter described.

Returning now to the mode select signal 367, mentioned briefly above with respect to the multiplexer 228, attention should be directed to Figures 5A and B and, more particularly, to the WRITE Request Logic 360 therein, which comprises a pair of flip-flops 362 and 364 and an AND gate 366 connected to a multiplexer 370. The mode select signal 367 may be seen to be the output signal from AND gate 366 which serves to synchronize production of the mode select 367 with the timing signal LCLK during time intervals in which data is not to be read for display. Accordingly, the flip-flops 363 and 364 are responsive to LCLK and to the absence of a DCLK pulse to enable the AND gate 366. A second input of the AND gate 366 is coupled to transistor 368 which disables the AND gate, as hereinafter described, to ensure that no data is written into memory in those addresses corresponding to video display positions lying outside the boundaries 14a to d and 22a to d (Figure 1). This latter function is provided by means of the transistor 368 being responsive to a vertical gating signal 332. The second input is additionally coupled to "freeze" circuit 372 (Figure 2B) which is actuable by operator request to prevent the writing of new data into memory.

The mode select signal 367 is additionally applied to a multiplexer 370 respectively to produce one of two possible WRITE enable signals WEO, WE1 which, by reference to Figure 3, may be seen as being applied to the memory matrix 111 in order to cause the writing of the data held in the latch 110 at the address specified by the WRITE address counter outputs 112a to h.

Having thus stored the echo information in memory, discussion is next turned to the manner by which the data

10

is read from memory for video display. Turning first to
the READ address, the object is to read the data associated
with successive video elements of each video line on a
line-by-line basis. As shown in Figure 2, and in more
detail in Figures 4A and 4B, the READ address circuitry
comprises the counter-enabling logic 200 and READ address
counters 226.

As shown in Figures 4A and 4B, the counter-enabling
circuitry comprises a flip-flop 210 coupled at its inverted
output to one input of an AND gate 216, and at its noninverted output to the LOAD terminal of a counter 212.
The counter 212 is coupled through an inverter to the
CLEAR terminal of the flip-flop 210. The inverted
output of the flip-flop 210 is additionally coupled to
the triggering input of the AND gate 216. The AND gate
216 is, in turn, coupled to the DATA terminal of a flip-
flop 220 which is clocked by the timing signal DCLK to
enable the READ counters. Thus, whilst the data
has been written into memory at each LCLK pulse, during
the time intervals between these pulses, the disclosed
system functions to read data out of memory for video
display at a rate determined by DCLK, with the memory being
accessed in a predetermined sequence to read out the
contents of the memory during each video line.

The counter-enabling circuitry defines the time
"window" during which the video beam is within the
display boundaries 14b and d; during this time the data
for a video line is accessed from memory. The commencement
of the "window" is referenced to the commencement of the
video line by a horizontal drive signal HDR produced at
a central processing unit to indicate the retracting of
the video scan beam. Accordingly, the window commences
after a brief delay to enable the displayed A-trace to
be spaced from the left edge of the video screen, as
shown in Figure 1A. To initiate the video window, the
flip-flop 210 is responsive to the horizontal drive signal

0001933

HDR to load the counter 212. The counter 212 is thereby loaded with a preselected number and counts towards its terminal count. The carry-signal 213 consequently generated clears the flip-flop 210 to initiate the window via an enabling signal 214 at one input of the AND gate 216. The signal 214 is additionally supplied to the triggering input of a one-shot multivibrator 218, which undergoes an output state change in a direction which enables the AND gate 216. The multivibrator 218 maintains the state change for a predetermined period of time, as determined by potentiometer R1, and thereafter returns to its initial state, thereby disabling the AND circuit 216, signifying the termination of the window. The flip-flop 220 thereafter functions to synchronize the commencement and termination of the window with the occurrence of DCLK. The output 22 of flip-flop 220 is a horizontal gating signal. When in the normal state, the signal 222 loads the READ address counters 226 with a predetermined number. When signal 222 changes state, the counters 226 increment at a rate determined by DCLK from the loaded value, until the termination of the window, at which time the signal 222 returns to its normal state, and the counters 226 are reloaded. The output count serves as the read address signal to the memory.

Turning next to Figure 2 for a general overview and to Figures 5A and 5B for a more detailed appreciation data retrieving circuitry will now be described. The data output signals 114 from the addressed memory location are applied as shown in Figure 5A to the input terminals of a multiplexer 310. It may be noted that the output signals from the right side of the matrix 111 (Figure 3) are applied to one bank of multiplexer inputs while the data from the left side of the memory matrix 111 is applied to the second bank of multiplexer 310 inputs. The multiplexer 310 is responsive to select signal 227 which is produced by the READ counters 226 as part of the memory address.

It may be helpful to remember that the output signal 312 from the multiplexer 310 represents quantized sampled signal levels of the returning echo reflections, where the sampling rate was determined by LCLK. If the contents of the memory are read out during every video line in the sequence in which they were stored and converted to an intensity modulated signal in a format compatible with the raster scan of the video beam, a video display of the A-trace is produced. Turning briefly to Figure 1, it may be appreciated that each video line of the graphically displayed A-trace will comprise both light and dark elements. For example, each point below the boundary of the graph may appear as a white video element, with the background appearing black. Since the ordinate of the A-trace represents the amplitude of the reflection, it is obvious that the intensity (i.e. black or white) of each picture element in a video line will depend upon whether the amplitude of the pulse reflection at the return time represented by the picture element's abscissa value is greater or less than the ordinate value associated with the particular video line. Accordingly, the data signal 312 from the multiplexer 310 is applied to the inputs of a comparator 314 and compared to threshold levels 315 produced by video line counters 320. The line counters 320 function to produce threshold levels corresponding to the ordinate values associated with the video lines. In the described embodiment, the comparator output 316 will produce a white picture element if the signal 312 is greater than the threshold 315, and will produce a black picture element if the signal 312 is less than the threshold 315. Consequently the area under the A-trace will contrast with the background. Naturally, the relationship may be reversed so that the area under the A-trace of Figure 1A would appear black against a white background.

With reference to Figures 5A and B, the line counters 320 are responsive to a vertical drive signal

0001933

VDR (from the video monitor) that signifies the vertical retrace of the video scanning beam. The signal VDR triggers delay device 326, a one shot multi-vibrator, the output signal 324 of which enables the counters 320 to count from a loaded value towards a terminal count when clocked by the signal HDR, indicative of the horizontal retrace of the video scanning beam. The counters 320 are configured to increment once every video line and to change output every eight video lines. Upon reaching this terminal count, the counters 320 are disabled by "carry" signal 328.

The A-trace converter includes circuitry to ensure that the data is displayed within the display portion of the video screen defined by the boundaries 14a to c (in Figure 1A), i.e. circuitry which produce vertical and horizontal gate signals. The horizontal gate signal 222 has been described above. The vertical gate signal 332 is produced a finite time interval after the vertical retrace of the video beam to ensure that the A-trace is displayed between the boundaries 14a and c. The "carry" signal 328 from the line counter 320, signifying the completion of the window in the vertical direction, is accordingly coupled, along with the time-delayed vertical drive pulse 324 which indicates the initiation of the window vertically, to respective inputs of an AND gate 330 producing the signal 332.

The output signal 316 from the comparator 314 is coupled with the horizontal gate signal 222 and the vertical gate signal 332 to respective inputs of an AND gate 334, which produces, as its output, a properly timed video beam modulating signal 338.

It may be appreciated that the original data signal was written into memory at a clock rate corresponding to fixed incremental depths into the examined body. Therefore each clock pulse on READ also corresponds to that depth and depth calibration is accordingly maintained

0001933

regardless of the DCLK rate; a different DCLK rate will accordingly result in the display of a different body depth along the horizontal direction, thereby creating a change in the scale factor.

As indicated earlier in this disclosure, the video display of the A-trace lends itself to operator-actuable video calipers which may be utilized to accurately measure the displayed distance into the body of the discontinuity-generating reflections as well as the difference in return times of a pair of discontinuities. It may be appreciated that since each DCLK pulse corresponds to a fixed distance,, counting these pulses enables one to measure distances.

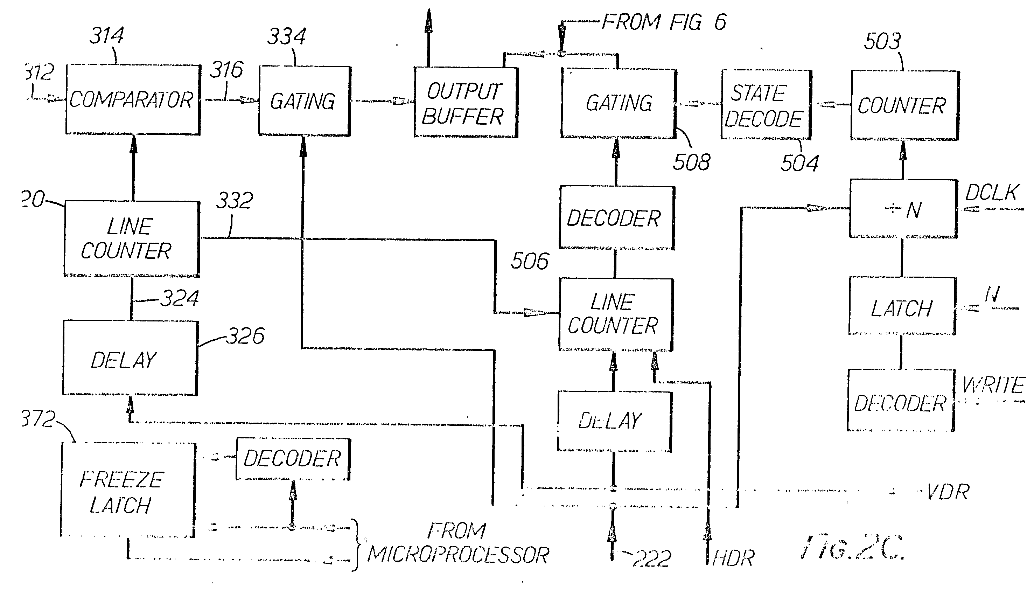

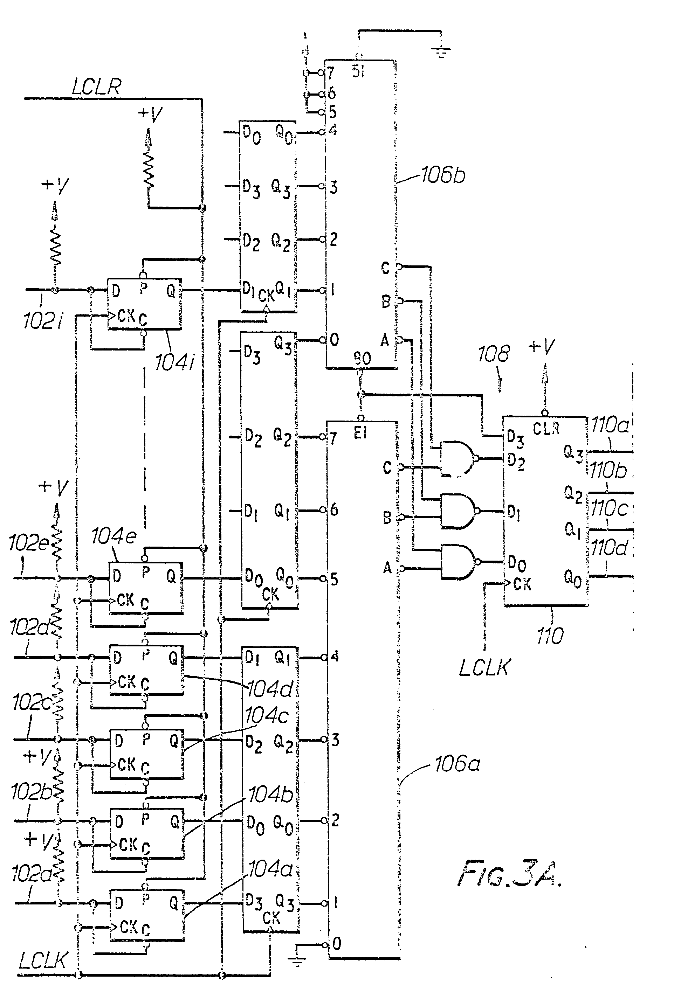

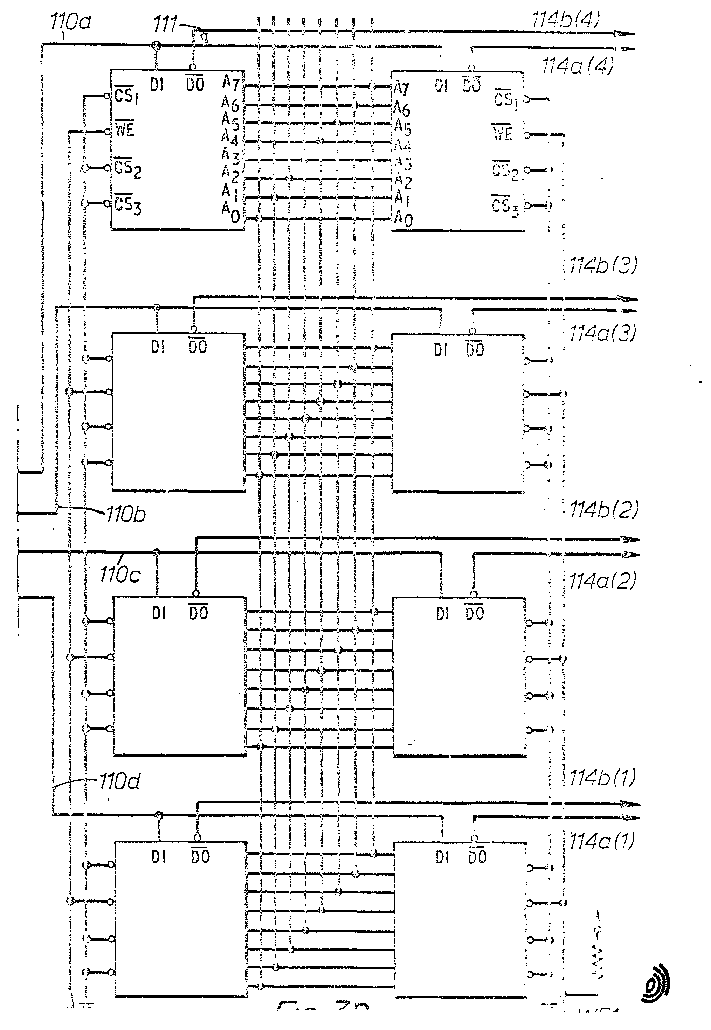

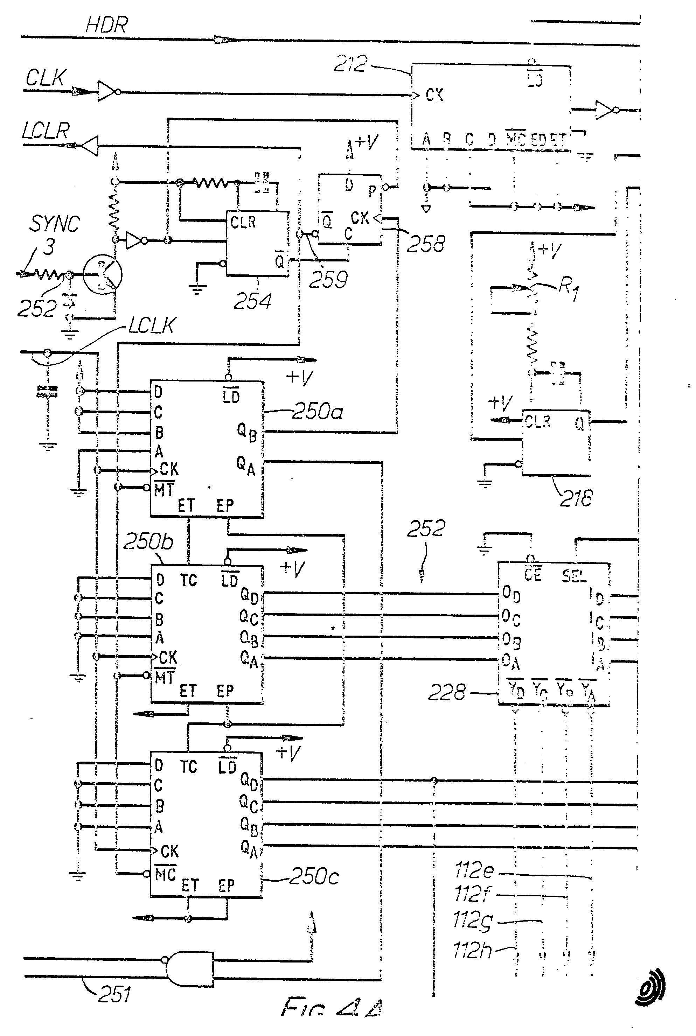

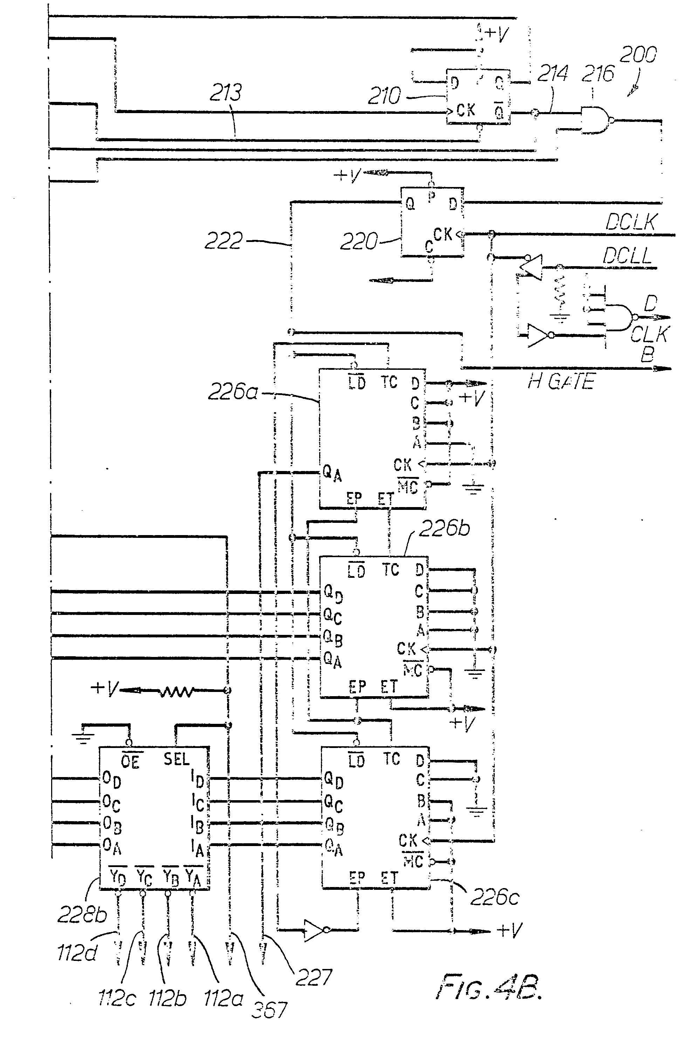

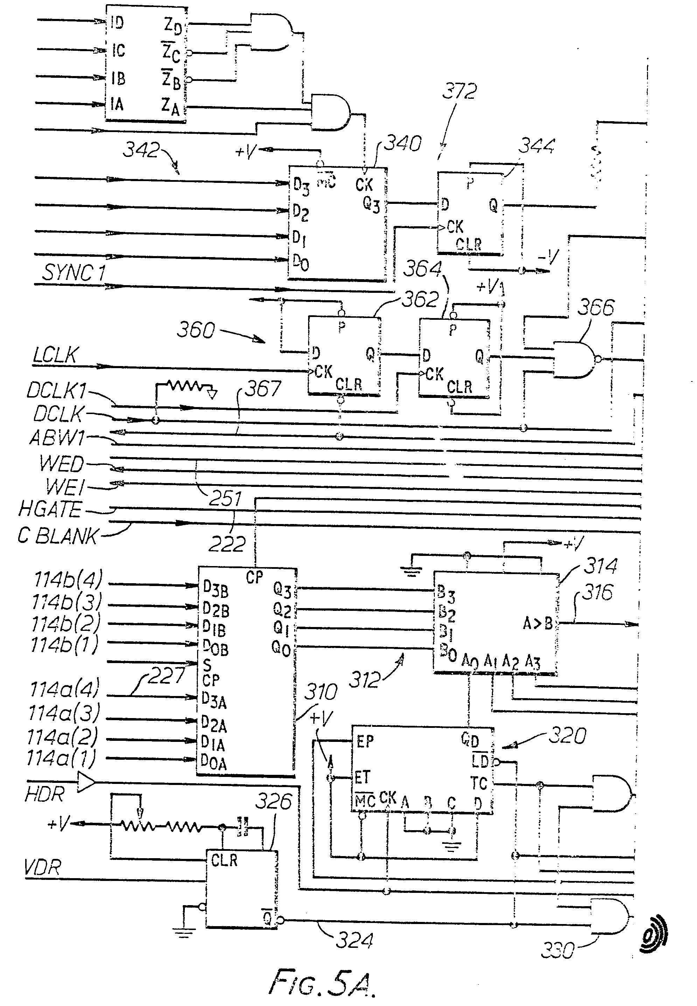

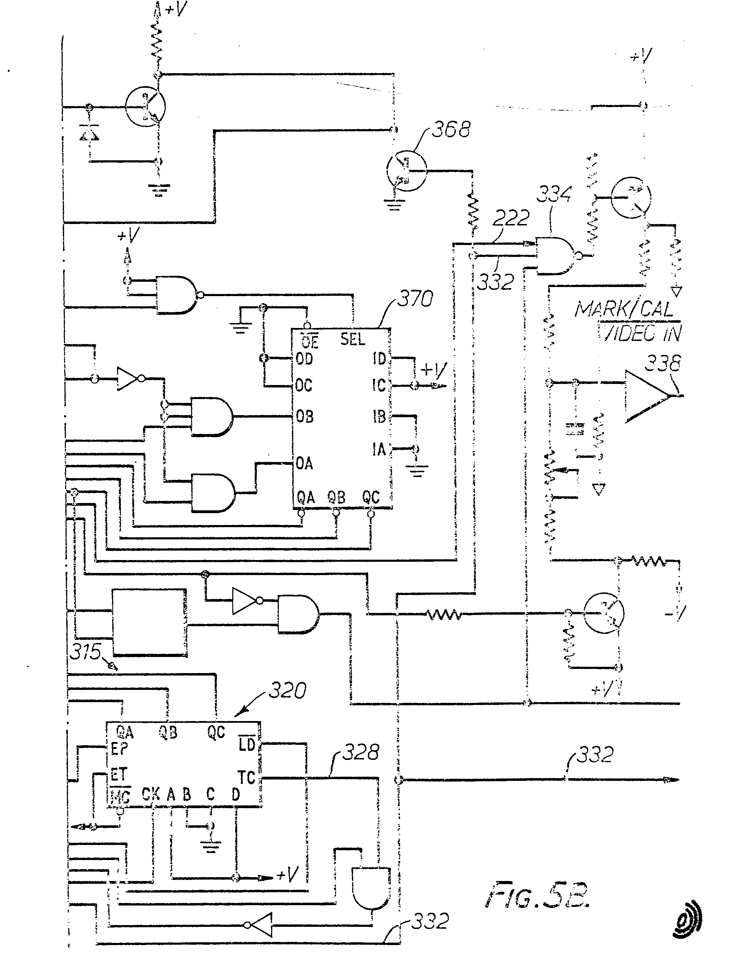

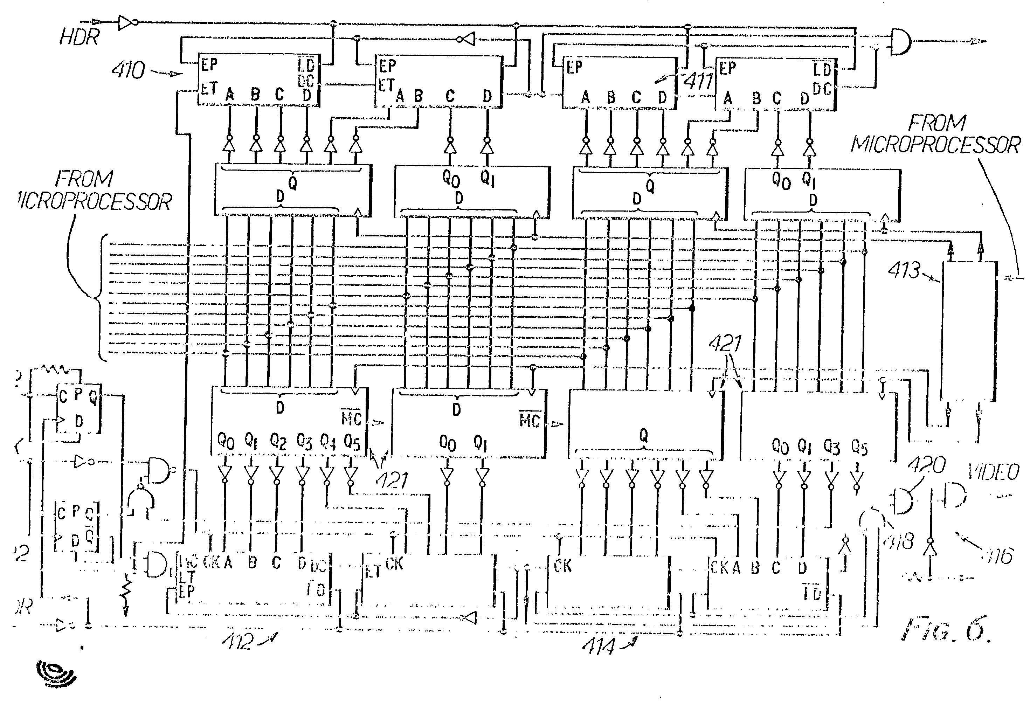

Figure 6 schematically illustrates the circuit for producing the caliper. The horizontal and vertical gate signals 222 and 332 pass through enabling logic which produce an enabling signal, synchronized with DCLK, to a two-stage counter 412. The counter 412 is a caliper delay counter which provides a variable delay from the beginning of the A-trace to the beginning of the caliper so that the caliper may be moved along the abscissa of the A-trace as desired by the operator. The counter 412 is incremented by DCLK, which is synchronized with the commencement of the video line, and enables the caliper-producing circuitry at its terminal count. The caliper producing circuitry comprises a caliper spacing counter 414 (two stages) which, as indicated hereinbelow, counts a variable number corresponding to the width of the caliper. The "carry" signals 413 and 415 from the counters 412 and 414 are coupled into an output stage 416.

The delay produced by the counters is determined by variable preloaded counts which are derived by the micro-processor in accordance with pre-programmed instructions in response to the wishes of the operator and are loaded in latches 421. The counters are loaded

0001933

in response to the horizontal retrace signal HDR at the commencement of each video line at the same initial point, and the delay counter begins counting at the start of the A-trace display time indicated by gate signal 222. Upon reaching the terminal count, the count 412 is disabled by its "carry" output 413 which additionally enables the counter 414 and enables the caliper display. The counter 414 now counts toward its terminal count. Upon reaching that count, it is disabled by its "carry" output 415, which also disables the caliper display.

The "carry" outputs 413, 415 from the counters 412, 414 are coupled to respective inputs of an AND gate 418 to produce a "window" during which time the caliper is to appear. The output of the gate 418 is similarly coupled to respective inputs of a second AND gate 420 along with a micro-processor signal indicative of an operator-induced "caliper request". As an added feature, the caliper-producing circuitry is overridden by the A-trace circuitry so that the caliper video will not appear where the A-trace video is.

The counters 412, 414 hold their terminal counts state until the next load pulse HDR, at which time they are reloaded and the counting sequence commences once again.

Similar latches and counters 410 and 411 enable the time gain control curve to be displayed . Selection between counters 410, 411 and 412, 414 is made by a selection circuit 413 receiving control data from the microprocessor.

Finally, Figure 2C also shows circuitry for producing the comb display of Figures 1A to 1C.

A "write" command signal comes from the microprocessor to a decoder 500 which enables a latch 501 to receive a value N defining the number of pulses/mm, from the microprocessor. This value N goes to a divide-by-N circuit 502 receiving DCLK clock pulses and an enabling signal which is the horizontal gating signal defining the horizontal start of the comb. The output of circuit 502 is supplied to a counter 503 which determines in conjunction with a decoder 504 the two types of mark in the comb, i.e. a long mark and a short mark.

A delay device 505 receives VDR from the microprocessor to define the vertical retrace and introduces a delay to define the top of a "window" for the comb. Line counter 506 then counts the necessary lines for the comb. Gating circuit 507 produces a corresponding video signal that is supplied to output gating 508.

0001933

CLAIMS:

1. An ultrasonic diagnostic video A-trace display system for use with ultrasonic diagnostic equipment of the type including means for launching an ultrasonic pulse into a body to be examined, means for detecting emerging pulse reflections from the body and responsive thereto to produce respective amplitude-indicative electrical signals, and video display means including means for repeatedly scanning a plurality of generally parallel video lines of the display means to define a multi-lined video field,

the video A-trace display system being characterised by:

memory means (111) having uniquely addressable locations for storing digital values;

means (250) for storing in a predetermined succession of locations of the memory means respective digital values indicative of pulse-reflection amplitudes at respective time-intervals subsequent to the launching of a pulse; means (200, 226) responsive to the initiation of a video line for accessing successive memory locations in a sequence which permits retrieval of the digital values in a reconstructed time-phase relationship; means (320) for producing a reference signal (315) having a value which is substantially proportional to the position within the video field of the video line being scanned; and

means (300) for comparing the digital values accessed with the reference signal to produce video signals respectively indicative of whether the successive digital values are greater than or less than the reference signal.

2. A video display system according to claim 1 and including means for producing a timing signal (DCLK), the memory access means (200, 226) including counter means (226) arranged to be incremented at a rate determined by the timing means to count from an initially preloaded value towards a terminal count for producing address signals for the memory means, and means (200) responsive

to the commencement by the video scan of a new video line to preload the counter means (262), the count between the initial and terminal values being related to the scan time of the video line so as to address during the scanning of the video line the memory locations containing the data to be displayed.

3. A video display system according to Claim 2, wherein the counter means (226) is adapted to address all the memory locations containing data to be displayed during the scanning of each video line in the A-trace field.

4. A video display system of Claim 1, 2 or 3 and including means (200) responsive to the commencement of each video line for producing a gate signal delayed therefrom, the gate signal being of shorter duration than the time remaining at its initiation for the video beam to complete the video line, and .

means (220) responsive to the gate signal for producing an enabling signal for the memory access means, whereby horizontal boundaries to the trace are produced.

5. A video display system according to Claim 4, for a video display means including means for scanning in a raster pattern, the gate signal producing means including means (210) for producing a bistable output signal, one of the states serving as a horizontal drive signal indicative of the horizontal retrace of the video scanning, bistable signal producing means (218) responsive to the horizontal drive signal to produce a time-delayed state change of said shorter duration, and logic gate means (216) responsive to the horizontal drive signal and the time-delayed signal to produce the enabling signal.

6. A video display system according to any one of the preceding claims and comprising first timing means responsive to the launching of ultrasonic pulses

0001933

to produce a first series of timing pulses (LCLK), second timing means responsive to the commencement of video lines to produce a second series of timing pulses (DCLK), means (104) responsive to the first timing pulses to sample the amplitude-indicative signals (102) at successive discrete times subsequent to ultrasonic pulse launching to produce corresponding digital values for the memory means, the storing means (250) being responsive to the first timing pulses (LCLK) for successively accessing the memory locations to permit storing of the successively produced digital values in respective locations, and the means (200,226) for accessing successive locations being responsive to the second timing pulses (DCLK) for successively accessing at least a portion of the locations accessed by the storing means (250), said portion being accessed during each video line scan.

7.    An ultrasonic diagnostic video A-trace display system for use with ultrasonic diagnostic equipment of the type including means for launching an ultrasonic pulse into a body to be examined, means for detecting emerging pulse  reflections from the body and responsive thereto to produce respective amplitude-indicative electrical signals, and video display means including means for repeatedly scanning a plurality of generally parallel video lines of the display means to define a multi-lined video field,

the video A-trace display system being characterised by:

first timing means responsive to the launching of an ultrasonic pulse for producing a first series of timing pulses (LCCK);

second timing means responsive to the commencment of a video line to produce a second series of timing pulses (DCLK);

memory means (111) having uniquely addressed locations for storing digital values;

4                          0001933

means (104) responsive to said first timing means
to sample the amplitude-indicative signals at successive
discrete times subsequent to the pulse launching and to
apply the successively sampled signals as digital data to
the memory means (111);

storing means (250) responsive to the first timing
means for successively accessing the address locations
to permit the storing of the successively sampled data
in respective memory locations;

means (200,226) responsive to the second timing
means for successively storing means, said portion being
accessed during each video line scan, and means (300)
responsive to the accessed data values for producing
video signals.

8. A video display system according to Claim 7
including counter means (226) responsive to the pulses
of the second series of pulses (DCLK) to count from an
initial value to a terminal value, the count value
being uniquely related to a respective memory address, and
means (200) responsive to the initation of each video line
to load the counter means (262) with the initial number,
whereby the counter means cycles through the addresses
containing the displayed information during each video
line.

9. A video display system according to claim 6
or 7 and including first counting means (250) incremented
by the first series of pulses (LCLK) from an initially
loaded count value to a termination count value, to
produce WRITE address signals, second counting means
(226) incremented by the second series of pulses (DCLK)
from an initially loaded count value to a termination
count value to produce READ address signals, multiplexing
means (228) selectively coupling the READ and WRITE address
signals to the memory means (111) in response to respective
states of a bistable mode signal, and means (360) for
producing the bistable mode signal, including means (362) responsive to the first series of timing pulses (LCLK) to produce a first request signal, means (364) responsive to the first request signal to produce a second request signal at an appropriate time relative to a pulse of the second series, and means (366) responsive to a subsequent pulse of the second series to couple the WRITE address signals to the memory means.

10.    A video display system  according to any one of the preceding claims and including  means responsive to the commencement of each video line and synchronized with the scanning thereof by the video scan to sequentially apply the successively retrieved data, to the means (300) for producing video signals, at a rate by which the last-displayed value is applied substantially proximate to the end of the video line.

FIG. 1A.

14b  14a  14d

18  18  12

16

14c

10

FIG. 1B.

12  22d

22a  22b  22c

FIG. 1C.

W  26

12

24

28

1/11

0001933

RECEIVER

33

RF IN

RF IN

COMMON

34

35

36

38

102

37

100

FROM
MICROPROCESSOR

FIG.2A.

Fig.2B.

11/33

0001933

FIG. 2C.

5/11

0001933

FIG. 3A.

000'933

Fig. 7b

0001933

Fig. 44

FIG.4B.

9/11

0001933

FIG.5A.

0001933

MARK/CAL
VIDEO IN

FIG.5B.

Fig. 6.